# EUROPEAN PATENT APPLICATION

(11) **EP 3 449 810 A1**
(43) Date of publication of application: **06.03.2019**
(21) Application number: 18192654.4
(22) Date of filing: 05.09.2018
(51) Int. Cl.: A61B 3/10, A61B 3/117, G06T 7/00

(54) **OPHTHALMOLOGICAL IMAGE PROCESSING PROGRAM**

(30) Priority: 05.09.2017 JP 2017170697
(71) Applicant: Nidek Co., Ltd., Gamagori-shi, Aichi, 443-0038 (JP)
(72) Inventor: HAMAGUCHI, Koji, Gamagori, Aichi (JP); SHIMIZU, Kazunari, Gamagori, Aichi (JP); SHIBA, Ryosuke, Gamagori, Aichi (JP); HANEBUCHI, Masaaki, Gamagori, Aichi (JP); MURATA, Keiji, Gamagori, Aichi (JP)
(74) Representative: Hoefer & Partner Patentanwälte mbB

(57) **Abstract**

An ophthalmological image processing program, when executed by a processor of a computer, causes the computer to perform an acquisition step of acquiring an entire periphery image being a reflected image of an entire periphery of an ACA (Anterior Chamber Angle) of a subject eye as an imaging result of the ACA by an angle imaging apparatus, and acquiring three-dimensional OCT data including the ACA as an imaging result of an anterior ocular segment of the subject eye by an anterior ocular segment OCT, and an association step of associating the entire periphery image with the three-dimensional OCT data in regard to a positional relationship.

## Description

### TECHNICAL FIELD

This disclosure relates to an ophthalmological image processing program for processing an image of an anterior ocular segment.

### BACKGROUND

In glaucoma diagnosis, it is useful to observe an ACA (Anterior Chamber Angle) of a subject eye. In the related art, an observation of an ACA is performed with the naked eyes via a goniolens. However, in recent years, various imaging methods of imaging an ACA have been proposed.

For example, WO2015/180923 discloses a method in which illumination light is applied toward an ACA, and a reflected image of the ACA is captured on the basis of reflected light from the ACA. For example, JP-A-2011-147611 discloses a method in which OCT data of an ACA is acquired as information regarding a section of the ACA with using an optical interference tomography meter.

However, there is no disclosure of association of a reflected image of an ACA with information regarding a section thereof. Thus, for example, it is hard for an examiner to multilaterally understand a structure and a state of an ACA at a position desired by the examiner.

### SUMMARY

An object of the present disclosure is to provide an ophthalmological image processing program that enables to generate information useful for an examiner to understand a structure and a state of an ACA.

In order to solve the above-described problem, the present disclosure includes the following configurations.
(1) An ophthalmological image processing program, when executed by a processor of a computer, causing the computer to perform:
   an acquisition step of acquiring an entire periphery image being a reflected image of an entire periphery of an ACA (Anterior Chamber Angle) of a subject eye as an imaging result of the ACA by a first imaging apparatus, and acquiring sectional information of the ACA as an imaging result of an anterior ocular segment of the subject eye by a second imaging apparatus; and
   a association step of associating the entire periphery image with the sectional information in regard to a positional relationship.
(2) The ophthalmological image processing program according to the above (1),
   in which, in the acquisition step, the processor acquires, as the entire periphery image, a color image in which visible light including a plurality of different wavelength ranges is used as illumination light.
(3) The ophthalmological image processing program according to the above (1) or (2),
   in which the second imaging apparatus is an OCT apparatus that acquires OCT data of the anterior ocular segment,
   in the acquisition step, the processor acquires three-dimensional OCT data of the anterior ocular segment as the sectional information, and
   in the association step, the processor associates the three-dimensional OCT data with the entire periphery image in regard to a positional relationship.
(4) The ophthalmological image processing program according to the above (3),
   in which, in the acquisition step, the processor acquires three-dimensional motion contrast data including a blood vessel region of the anterior ocular segment as the three-dimensional OCT data, and
   in the association step, the processor associates the three-dimensional motion contrast data with the entire periphery image in regard to a positional relationship.
(5) The ophthalmological image processing program according to the above (4),
   in which, in the acquisition step, the processor further acquires three-dimensional intensity OCT data corresponding to the three-dimensional motion contrast data, and
   in the association step, the processor associates the three-dimensional intensity OCT data with the entire periphery image to indirectly associate the three-dimensional motion contrast data with the entire periphery image.
(6) The ophthalmological image processing program according to any one of the above (3) to (5),
   in which, in the association step, the processor performs a matching process between a three-dimensional image based on the three-dimensional OCT data and the entire periphery image to associate the three-dimensional OCT data with the entire periphery image in regard to a positional relationship.
(7) The ophthalmological image processing program according to any one of the above (3) to (5),
   in which the first imaging apparatus projects and receives illumination light via an optical axis parallel to an angle division line in the ACA, and acquires the entire periphery image based on illumination light projected and received while changing a position of the optical axis with respect to a plurality of positions in the ACA entire periphery, and
   in the association step, the processor acquires an OCT front image viewed along the optical axis of the first imaging apparatus based on the three-dimensional OCT data, and associates the three-dimensional OCT data with the entire periphery image in regard to a positional relationship by performing a matching process between the OCT front image and the entire periphery image.
(8) The ophthalmological image processing program according to the above (1) or (2),
   in which the first imaging apparatus projects and receives illumination light via an optical axis parallel to an angle division line in the ACA, and acquires the entire periphery image based on illumination light projected and received while changing a position of the optical axis with respect to a plurality of positions in the ACA entire periphery,
   in the acquisition step, the processor acquires a three-dimensional image based on a plurality of sectional information in which positions of sections are different from each other, and acquires a two-dimensional image in a case of viewing the three-dimensional image along the optical axis of the first imaging apparatus, and
   in the association step, the processor associates the entire periphery image with the three-dimensional image by performing a matching process between the two-dimensional image and the entire periphery image.
(9) The ophthalmological image processing program according to the above (7) or (8),
   in which the matching process is an image process performed based on an interrelation between images.
(10) The ophthalmological image processing program according to the above (7) or (8),
   in which the matching process is pattern matching performed with using either of information regarding a shape of the ACA common to images and information regarding a feature part of the ACA common to images.
(11) The ophthalmological image processing program according to any one of the above (6) to (10),
   in which, in the acquisition step, the processor acquires, as the entire periphery image, a multi-focus image being in focus on a plurality of kinds of different feature parts in a single image.
(12) The ophthalmological image processing program according to the above (11),
   in which the multi-focus image is a combined image obtained through a combination of a plurality of the reflected images in which focus states of an imaging optical system in the first imaging apparatus are different from each other.
(13) The ophthalmological image processing program according to any one of the above (1) to (4),
   in which the first imaging apparatus further captures a first front image being a front image of the anterior ocular segment during capturing of the entire periphery image,
   the second imaging apparatus further captures a second front image being a front image of the anterior ocular segment during generation and acquisition of the sectional information, and
   in the association step, the processor associates the entire periphery image with the sectional information through the first front image and the second front image.
(14) The ophthalmological image processing program according to any one of the above (1) to (13), when executed by the processor of the computer, causing the computer to further perform:
   a display control step of simultaneously displaying the entire periphery image and a sectional image on a monitor in an aspect that the entire periphery image is associated with the sectional image based on the sectional information in regard to a positional relationship.

### BRIEF DESCRIPTION OF DRAWINGS

Fig. 1 is a diagram illustrating a summary of an ophthalmological system of an embodiment.
Fig. 2 is a diagram illustrating a summary of an angle imaging apparatus included in the ophthalmological system.
Fig. 3 is a diagram illustrating a reflected image of an ACA captured by the angle imaging apparatus.
Fig. 4 is a diagram illustrating an entire periphery image obtained through a collage of reflected images captured at different imaging positions.
Fig. 5 is a diagram illustrating a summary of an anterior ocular segment OCT included in the ophthalmological system.
Fig. 6 is a diagram illustrating a sectional image of an ACA captured by the anterior ocular segment OCT.
Fig. 7 is a diagram illustrating a summary of a computer included in the ophthalmological system.
Fig. 8 is a flowchart illustrating an operation in the computer.
Fig. 9 illustrates a first display example in an aspect of associating an entire periphery image with a sectional image each other.
Fig. 10 illustrates a second display example in an aspect of associating an entire periphery image with a sectional image each other.

### DETAILED DESCRIPTION

### [Summary]

An embodiment of the present disclosure will be described with reference to the drawings. Hereinafter, a description will be made of the embodiment regarding an ophthalmological system, an ophthalmological device, an ophthalmological image processing program and method, and an ophthalmological device control program and method. Items classified by the following < > may be used independently or in association therewith.

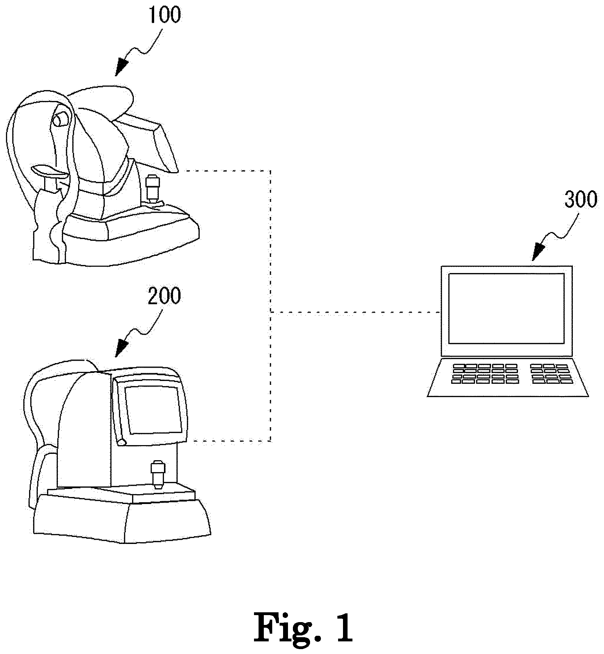

An ophthalmological system described below includes at least a first imaging apparatus, a second imaging apparatus, and a computer (refer to Fig. 1). The first imaging apparatus and the second imaging apparatus are modalities which are different from each other, and image an ACA (Anterior Chamber Angle) of a subject eye in different imaging methods.

### <First Embodiment>

A description will be made of a first embodiment. In the first embodiment, the first imaging apparatus and the second imaging apparatus independently image an ACA (and a periphery thereof). Imaging results by the respective imaging apparatuses are associated with each other by the computer, and are displayed on a monitor.

### <Schematic configuration of system>

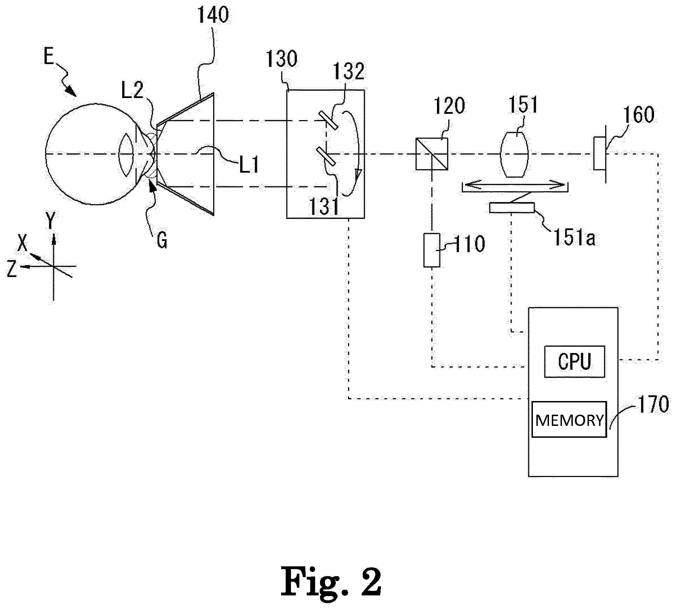

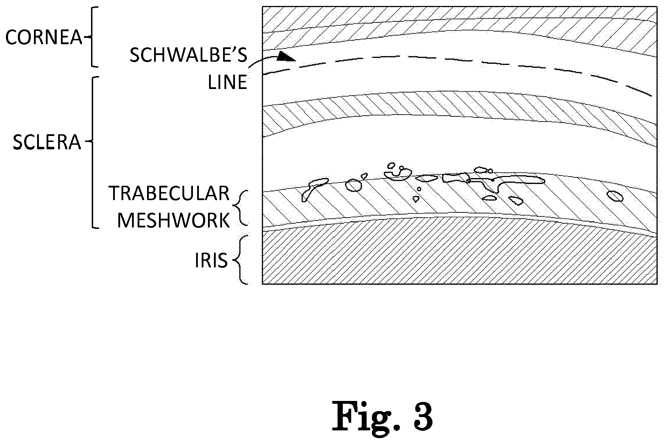

The first imaging apparatus captures reflected images of the ACA with respect to the entire periphery (in other words, 360 degree angle around an eye axis) of the ACA. For example, the first imaging apparatus has an optical axis which is tilted with respect to the eye axis, and captures a reflected image of the ACA as a result of projecting and receiving illumination light along the optical axis (refer to Figs. 2 and 3). Specifically, the illumination light is emitted and received along the optical axis which is parallel to an angle division line (a line which divides the cornea from the iris) of the ACA. For example, an image in which the ACA is projected to a plane intersecting the angle division line is obtained as a reflected image. The first imaging apparatus may have a configuration (imaging optical system) in which light is applied to a surface tissue of the ACA in the entire imaging range, and reflected light from the surface tissue is received by a light receiving element. The first imaging apparatus may have a configuration in which scanning with illumination light is performed in the imaging range. A reflected image may be a monochromatic image and may be a color image. The color image is obtained on the basis of illumination light containing a plurality of different wavelength regions. For example, the color image is an image having at least three color components (for example, color components such as red, blue, and green). The color image is useful to check a function related to aqueous humor outflow. For example, the extent of pigmentation of the trabecular meshwork formed in one of principal aqueous humor outflow paths can be checked from the color image.

In the present embodiment, the "imaging range" is a region where is imaged on the basis of, for example, an imaging trigger which is input once. Hereinafter, unless particularly otherwise mentioned, a description will be made assuming that an imaging range in the first imaging apparatus is a part of the entire periphery of the ACA. However, this is only an example, and an imaging range in the first imaging apparatus may be the entire periphery of the ACA.

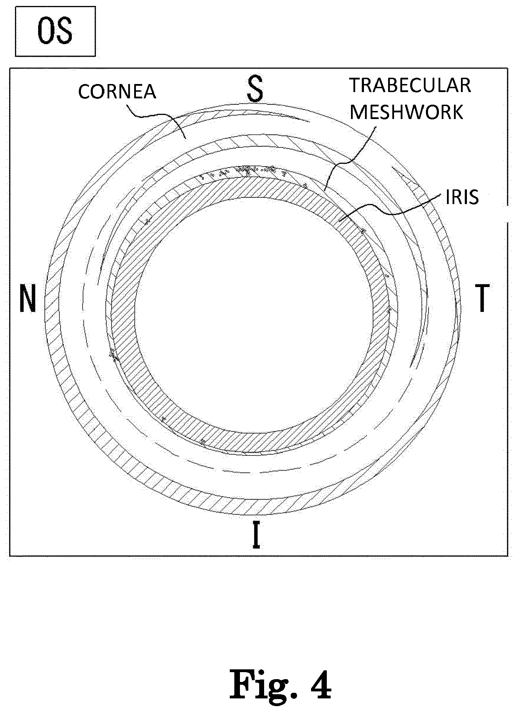

The first imaging apparatus may image the entire periphery of the ACA by performing imaging while changing a position (that is, an imaging position) where illumination light is emitted and received in the entire periphery of the ACA to the vicinity of the eye axis. In this case, for example, imaging results of the entire periphery of the ACA in the first imaging apparatus may be output as a plurality of ACA images captured at different imaging positions. Of course, a single image having the entire periphery of the ACA as an imaging range, that is the entire periphery image, may be output as an imaging result in the first imaging apparatus (refer to Fig. 4).

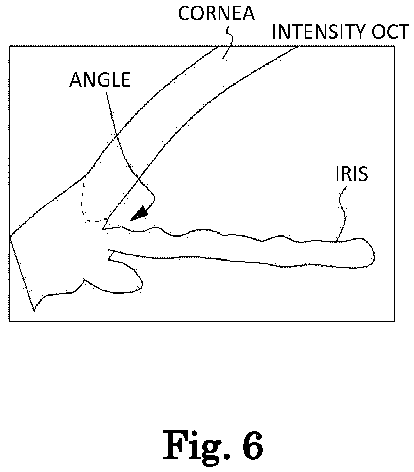

The second imaging apparatus acquires sectional information of the ACA as an imaging result of the anterior ocular segment of a subject eye. Various apparatuses which can image a section of the ACA may be used as the second imaging apparatus. For example, the second imaging apparatus may be an OCT (optical coherence tomography) apparatus, a Scheimpflug camera or a UBM (ultrasound biomicroscopy). The sectional information may be a sectional image (refer to Fig. 6) indicating any section of the ACA or may be data before being generated as an image. The sectional information acquired by the second imaging apparatus may be three-dimensional information indicating a three-dimensional structure of the ACA. In this case, sectional information regarding a plurality of different sections are acquired by the second imaging apparatus, and the three-dimensional information is generated on the basis of the plurality of acquired sectional information. The three-dimensional information may be, for example, a three-dimensional image.

The computer processes an image of or information regarding the ACA acquired by each imaging apparatus, and performs display control or operation control for any imaging apparatus on the basis of a process result. In the present embodiment, programs executed by a processor of the computer include an ophthalmological image processing program which is stored in advance in a memory which can be accessed by the processor.

The computer may be an apparatus (ophthalmological apparatus) integrated with any imaging apparatus, and may be a separate apparatus. Each imaging apparatus and the computer may be connected to each other in a wired or wireless manner such that any data can be transmitted and received.

The computer outputs an entire periphery image and a sectional image to the monitor to be displayed on the monitor.

### <Acquisition of entire periphery image and sectional information>

The computer acquires an entire periphery image which is a reflected image of the entire periphery of the ACA as an imaging result of the ACA in the first imaging apparatus. The computer acquires sectional information of the ACA as an imaging result of the anterior ocular segment of the subject eye in the second imaging apparatus. The term "acquisition" mentioned here indicates either one or both of operations in which an entire periphery image and sectional information are stored in a memory which can be accessed from the computer, and an entire periphery image and sectional information stored in the memory in advance are read.

In a case where the computer is an apparatus separate from both of the first imaging apparatus and the second imaging apparatus, an entire periphery image or sectional information may be stored in a memory which can be accessed from the computer, through image transmission from each imaging apparatus. In a case where the computer is integrated with one of the first imaging apparatus and the second imaging apparatus, an entire periphery image or sectional information may be stored in a memory which can be accessed from the computer as an imaging result in the integrated imaging apparatus.

The entire periphery image is a reflected image having the entire periphery of the ACA as a range. The entire periphery image may be generated through a collage of a plurality of images in which imaging positions are different from each other in the entire periphery of the ACA. In a case where the first imaging apparatus and the computer are separate apparatuses, the collage may be performed by either one of the first imaging apparatus and the computer.

### <Acquisition of multi-focus image>

The computer may acquire an entire periphery image based on an image (for convenience, referred to as a "multi-focus image") which is in focus in a wide range. In this case, a multi-focus image which is focus on a plurality of kinds of different feature parts in a single image is preferably acquired. The feature parts mentioned here include, for example, a Schwalbe's line, the trabecular meshwork, the scleral spur, the ciliary band, and a lesion part. Of course, the entire periphery image may be an image which is in focus on the substantially entire region. Regarding focus states at respective positions in the multi-focus image, complete focusing is not necessarily required to be realized, and a slight deviation is allowed within a desired purpose range. For example, in a case where the multi-focus image is used for a matching process with another image, a focus state may be deviated from a complete focusing state within a range of accuracy which is allowed in the matching process.

The first imaging apparatus captures a reflected image of the ACA by projecting and receiving illumination light along the angle division line of the ACA, and thus there is a great height difference with an ACA tip as a bottom in an imaging range of a subject including the ACA. However, in the multi-focus image, even if there is a height difference between tissues of the ACA, defocus in each part is favorably suppressed, and thus an examiner can favorably observe the ACA.

In the present embodiment, the multi-focus image may be a combined image obtained through a combination (collage) of a plurality of reflected images which are captured in a state in which focus states of the imaging optical system in the first imaging apparatus are different from each other. In this case, the first imaging apparatus performs imaging such that a plurality of ACA reflected images in which focus states are different from each other at respective positions in the ACA entire periphery. A region used for the collage in each of the ACA reflected images may be determined on the basis of focus evaluation information indicating a focus state of each image region included in each ACA reflected image. An image region of which a focus state is most favorable is selected from a plurality of ACA reflected images in which focus states are different from each other at each position on the ACA, and image regions selected for the respective positions are subjected to a collage, so that an ACA reflected image which is in focus in a wide range is obtained. Such a collage is performed on the ACA entire periphery, and thus an entire periphery image which is in focus in a wide range can be formed. The collage may be performed by either one of the first imaging apparatus and the computer.

In a case where a depth of field in the imaging optical system of the first imaging apparatus is sufficiently large, an entire periphery image which is in focus in a wide range may be obtained as a result of imaging at respective positions on the ACA entire periphery in a predetermined focus state. In this case, the depth of field in the imaging optical system of the first imaging apparatus is also preferably large for a height difference between different kinds of feature parts.

### <Association of entire periphery image with sectional information>

In a case where an entire periphery image and sectional information related to an ACA of a subject eye are acquired in advance, the computer may associate the entire periphery image with the sectional information in regard to positional relationship.

### <Association by matching process>

For example, the computer may associate an entire periphery image with three-dimensional information in regard to a positional relationship by performing a matching process (image matching).

For example, the computer may acquire a two-dimensional image on the basis of three-dimensional information, and match the acquired two-dimensional image with an entire periphery image. The two-dimensional image may be acquired by processing three-dimensional information which is acquired as an imaging result in the second imaging apparatus. Since an acquisition position of each piece of sectional information configuring the two-dimensional image is known, the acquisition position of each piece of sectional information can be specified on an entire periphery image on the basis of a result of matching between the two-dimensional image and the entire periphery image. In other words, the entire periphery image and the sectional information are associated with each other in regard to a positional relationship.

The two-dimensional image may be a front image in which a three-dimensional image based on the three-dimensional information is viewed along the optical axis of the first imaging apparatus. The two-dimensional image may be an integrated image in which three-dimensional images are integrated in an optical axis direction of the first imaging apparatus, may be an image obtained by extracting a plane at any position in the optical axis direction of the first imaging apparatus, may be a projection image in which a surface layer of a three-dimensional image is projected to a plane orthogonal to the optical axis of the first imaging apparatus, and may be other two-dimensional images. Such a two-dimensional image can be expected to have a high interrelation with a corresponding region in an entire periphery image. Thus, there is an advantage in that an association of an entire periphery image with sectional information by performing matching is performed with higher accuracy. In order to obtain a two-dimensional image, information regarding an angle between a depth direction of sectional information and the optical axis direction of the first imaging apparatus is necessary, but this may be acquired in advance as a constant based on design values between the respective imaging apparatuses.

An entire periphery image may be generated as a three-dimensional image, and a matching process may be performed on the three-dimensional entire periphery image and a three-dimensional image based on three-dimensional information. Undulations of a subject in an entire periphery image have an interrelation with focus evaluation information at respective positions on the entire periphery image, and thus the entire periphery image may be generated as a three-dimensional image through the use of a plurality of entire periphery images in which focus states are different from each other during imaging. However, a method of generating a three-dimensional image is not necessarily limited thereto.

The matching mentioned here may be an image process (for example, a phase restricting correlation method) based on phase information of each image, and may be pattern matching. In the pattern matching, information regarding a shape of the ACA or information regarding a feature part of the ACA may be extracted in terms of a pattern so as to be used for matching, and information regarding a feature (a luminance distribution or the like) on an image may be extracted in terms of a pattern so as to be used for matching.

The information regarding a shape of the ACA may be information regarding a position and a shape of a boundary between the cornea and the iris, or the sclera and the iris, may be information regarding a position and a shape of an undulation of tissue, and may be information regarding a position and a shape of tissue which is formed in a ring shape substantially centering on the eye axis. As the tissue formed in a ring shape, the pupil, a Schwalbe's line, the trabecular meshwork, the scleral spur, or the ciliary band may be used. An orientation and a position of a major axis or radius of an ellipse of when any one of the tissues is elliptically approximated to the ellipse may be used as information regarding a position and a shape of the ACA. As the information regarding a feature part of the ACA, in addition to a Schwalbe's line, the trabecular meshwork, the scleral spur, the ciliary band, and a lesion part, information regarding a position and a shape of a pigmented part or the like may be used. As the information regarding a feature point, a pattern of the iris may be used. Among the information regarding a shape of the ACA or a feature point of the ACA, information common to an entire periphery image and an image (a two-dimensional image or a three-dimensional image) based on three-dimensional information may be used for the pattern matching.

In a case where an entire periphery image is a multi-focus image, the influence of defocus in the entire periphery image during matching is small, and thus an association of the entire periphery image with sectional information is easily performed with high accuracy.

### <Association of motion contrast data with entire periphery image>

In a case where the second imaging apparatus is an anterior ocular segment OCT, the computer acquires three-dimensional OCT data of the anterior ocular segment including the ACA, as three-dimensional information.

The computer may acquire three-dimensional motion contrast data which one kind of three-dimensional OCT data, and may associate the three-dimensional motion contrast data with an entire periphery image. The three-dimensional motion contrast data may be acquired by performing a process of calculating a plurality of OCT signals which are acquired for an identical part at different times. Motion contrast may be information obtained by understanding motion of an object (for example, a blood flow or a tissue change). Regarding a method of deriving three-dimensional motion contrast data, for example, JP-A-2017-006179 is referred to.

For example, the content described in the above <Association using matching process> may be applied to matching between three-dimensional motion contrast data and an entire periphery image. The matching may be performed with using a motion contrast image as a result of generating three-dimensional motion contrast data as an image.

The computer may additionally acquire three-dimensional intensity OCT data (one kind of three-dimensional OCT data) obtained by imaging the same range as that of three-dimensional motion contrast data, and may indirectly associate the three-dimensional motion contrast data with an entire periphery image by associating the three-dimensional intensity OCT data with the entire periphery image.

### <Association of entire periphery image with sectional information based on anterior ocular segment front image>

A method which is different from the above-described method may be used for an association of an entire periphery image with sectional information. As an example, there may be a method of using anterior ocular segment front images captured respectively by the first imaging apparatus and the second imaging apparatus.

In this case, each of the first imaging apparatus and the second imaging apparatus includes an anterior ocular segment imaging optical system which can capture an anterior ocular segment front image. The anterior ocular segment imaging optical system has, for example, an imaging surface conjugate to the anterior ocular segment, and captures an anterior ocular segment front image in an angle of view approximately including upper and lower eyelids of a subject eye. In each imaging apparatus, an anterior ocular segment front image may be captured simultaneously with each of a reflected image and sectional information of the ACA.

In the method described here, an imaging position of a reflected image and an acquisition position of sectional information which are estimated on the basis of imaging conditions in each imaging apparatus are corrected on the basis of an anterior ocular segment front image captured simultaneously therewith. As a result of correction, in each of the reflected image and the sectional information, a reference position for specifying a position in the anterior ocular segment matches between the anterior ocular segment front image and the sectional information (that is, at least the reference position is associated).

The pupil center may be used as the reference position. Other positions such as a cornea apex position may be used as the reference position. Specifically, deviation amounts from alignment reference positions at the time of capturing a reflected image of the ACA and at the time of imaging sectional information may be derived from anterior ocular segment front images which are respectively captured simultaneously with the reflected image and the sectional information. Relationships between deviation amounts from the respective alignment reference positions, and a deviation amount of an imaging position of the reflected image of the ACA and a deviation amount of an acquisition position of the sectional information may be acquired in advance through tests or the like. Therefore, an imaging position of a reflected image and an acquisition position of sectional information which are estimated on the basis of imaging conditions in each imaging apparatuses can be corrected on the basis of the deviated deviation amounts so as to be made to match reference positions. In a case where an entire periphery image is generated on the basis of reflected images in which imaging ranges are different from each other, the reflected images are subjected to a collage such that mutual reference positions match each other, and thus the entire periphery image associated with sectional information at the reference position can be obtained.

In the method mentioned here, the influence on an imaging position and an acquisition position due to a difference in a rotary amount of the eye between the time of capturing a reflected image and the time of acquiring sectional information may be corrected. Specifically, rotary amounts of the eyeball at the time of capturing a reflected image of the ACA and the time of imaging sectional information thereof may be derived from two anterior ocular segment front images which are respectively captured simultaneously with the reflected image and the sectional information of the ACA. A positional relationship between the reflected image (and the entire periphery image) and the sectional information is adjusted on the basis of the rotary amounts, and thus a correspondence relationship between a position on the entire periphery image and the sectional information can be specified with respect to each line of longitude passing through the reference position.

### <Display process>

The computer may simultaneously display an entire periphery image and a sectional image in an aspect in which the entire periphery image and the sectional image are associated with each other. For example, the entire periphery image and the sectional image may be displayed in an aspect in which one thereof is merged into the other image. As an example, the entire periphery image and the sectional image may be displayed in an aspect in which the entire periphery image is attached to an ACA surface layer in a three-dimensional image of the ACA on the basis of a plurality of sectional images.

In a case where an entire periphery image is a color image, color information of each part in the entire periphery image may be reflected in a position corresponding to each part at least on a three-dimensional image. In other words, a three-dimensional image which is colored on the basis of the color information of each part included in the entire periphery image may be displayed on the monitor along with the entire periphery image or alone.

In a case where a three-dimensional image is colored, a color predefined for each part of the ACA in the three-dimensional image may be added. In this case, each part of the ACA may be specified, for example, by analyzing the three-dimensional image.

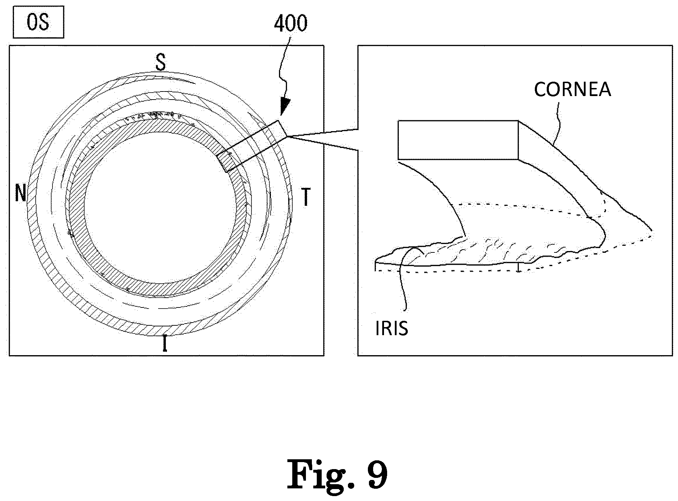

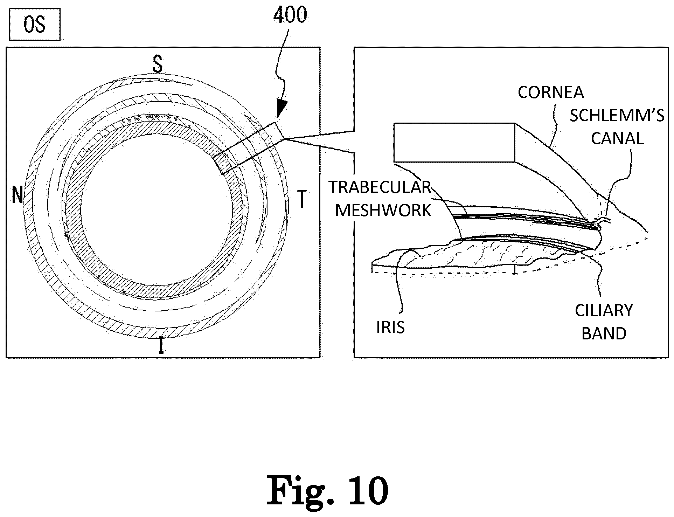

An entire periphery image and a sectional image may be individually displayed in separate regions on a screen, and an acquisition position of the sectional image may be displayed on the entire periphery image. For example, the acquisition position of the sectional image may be displayed by merging an index (graphic) indicating the acquisition position of the sectional image onto the entire periphery image. The acquisition position of the sectional image may be displayed by making image information such as luminance or a color of the acquisition position of the sectional image on the entire periphery image different from that of surroundings. Of course, the acquisition position of the sectional image may be displayed in other methods (refer to Figs. 9 and 10).

The computer may receive an operation of designating a part (a point, a section, or a region) on an entire periphery image in a state in which the entire periphery image is displayed on the monitor, so that a sectional information corresponding to the designated part is displayed simultaneously with the entire periphery image on the monitor.

For example, the extent of closure of the ACA and a state of the Schlemm's canal which are referred to in classification of glaucoma and grading of angle closure glaucoma can be more easily checked from the sectional image than from the entire periphery image, and, for example, the extent of pigmentation (in other words, the extent of clogging of the trabecular meshwork) or a lesion in the trabecular meshwork can be more easily checked from the entire periphery image than from the sectional image. For example, the entire periphery image and the sectional image are displayed in an aspect in which the entire periphery image and the sectional image are associated with each other, and thus it becomes easier for an examiner to multilaterally understand a structure and a state of the ACA at a desired position.

A display aspect in which a motion contrast image as a sectional image is associated with an entire periphery image is expected to be useful to examine a relationship between either blood vessel information or blood flow information around the ACA and glaucoma in the future. In this case, any of blood vessel density information, flow velocity information of a blood flow, and the like may be acquired by analyzing three-dimensional motion contrast data, and the motion contrast image and the entire periphery image may be displayed together.

### <Display of angle information of ACA>

The computer may display angle information indicating an angle of the ACA in at least a part of an entire periphery image on the monitor along with at least the entire periphery image. The angle information may be derived on the basis of corresponding sectional information. The angle information is information referred to, for example, in classification of glaucoma and grading of angle closure glaucoma. The angle information may be, for example, information indicating an angle with a numerical value, and may be information indicating an angle classified in each predefined range with characters or the like. In the present embodiment, angle information at different positions on the ACA entire periphery may be displayed simultaneously or in a switching manner.

### <Detection of tissue and feature part based on polarization characteristic data in ACA region>

Polarization characteristic data indicating a polarization characteristic of the ACA may be acquired on the basis of an imaging result in at least one of the first imaging apparatus and the second imaging apparatus. Return light of light applied to the ACA from each imaging apparatus is divided into light of a first polarization component and light of a second polarization component in which polarized light beams are different from each other, and polarization characteristic data is acquired on the basis of the light of the first and second polarization components. The polarization characteristic data may be an analysis result of a polarization characteristic, and may be at least any one of, for example, information regarding birefringence (birefringence information) of an ACA region, a degree of polarization uniformity (DOPU) of the ACA region, and information regarding polarization axis-orientation of the ACA region. A method of calculating such information is a well-known technique to a person skilled in the art, and thus a description thereof will be omitted.

A detection unit detects a feature part of the ACA on the basis of the polarization characteristic data. For example, a characteristic tissue included in the trabecular meshwork may be detected as a feature part.

A tissue detected as a feature part is not necessarily required to be included in the trabecular meshwork, and a tissue other than the trabecular meshwork may be detected as a feature part of the ACA. In this case, a tissue having a characteristic which is different from that of other parts of the ACA in one or more of various polarization characteristics such as the refractivity, the degree of polarization uniformity, and the polarization axis-orientation may be detected as a feature part. A specific example of such a feature part may be a filamentous tissue of the ACA, and may be a tissue of the ACA having a predetermined pigment. A tissue having a polarization characteristic may be excluded on the basis of polarization characteristic data, and remaining parts may be detected as feature parts. For example, since the Schlemm's canal is hollow, a hollow portion not having a polarization characteristic may be detected according to this method. The Schlemm's canal may be detected, for example, on the basis of polarization characteristic data.

An artifact present in a subject eye may be detected as a feature object on the basis of polarization characteristic data. The artifact may be, for example, a device implanted into a living body such as a glaucoma implant.

In a case where a feature tissue included in the trabecular meshwork is detected, the detection unit may detect the feature tissue with using a difference in a polarization delay amount from other tissues, caused by the birefringence of the trabecular meshwork. In this case, the trabecular meshwork (filamentous tissue) is detected on the basis of polarization characteristic data including information regarding a polarization delay amount at each position in the ACA.

A feature tissue included in the trabecular meshwork, detected as a feature part is not necessarily required to be the trabecular meshwork itself (filamentous tissue itself). For example, a pigmented part of the trabecular meshwork may be detected as a feature part. Depolarization (polarization scrambling) occurs in the pigmented part. Consequently, the pigmented part may be characteristically detected on the basis of the depolarization. The depolarization has an interrelation with the degree of polarization uniformity (DOPU), and thus the pigmented part may be characteristically detected on the basis of the degree of polarization uniformity. Therefore, in the present embodiment, the detection unit may detect the pigmented part on the basis of polarization characteristic data including depolarization or the degree of polarization uniformity at each position in the ACA. Of course, a feature tissue included in the trabecular meshwork may be a filamentous tissue of the trabecular meshwork and tissues other than the pigmented part.

As mentioned above, one or both of the filamentous tissue and the pigmented part of the trabecular meshwork may be detected on the basis of at least polarization characteristic data.

### <Setting of acquisition position of sectional information based on entire periphery image>

An entire periphery image captured by the first imaging apparatus may be used to set an acquisition position of sectional information in the second imaging apparatus. Herein, an operation of acquiring sectional information of a specific ACA tissue detected from an entire periphery image or a section corresponding to a position designated by an examiner is performed by the second imaging apparatus.

Anterior ocular segment front images respectively captured by the first imaging apparatus and the second imaging apparatus are used to specify a section for which sectional information is to be acquired in the second imaging apparatus. For convenience, an anterior ocular segment front image captured by the first imaging apparatus will be referred to as a first front image, and an anterior ocular segment front image captured by the second imaging apparatus will be referred to as a second front image. The second front image is an observation image used for alignment (and/or tracking) of a subject eye in the second imaging apparatus.

Each of the first imaging apparatus and the second imaging apparatus includes an anterior ocular segment imaging optical system which can capture an anterior ocular segment front image. The first front image which is captured simultaneously with any one of reflected images configuring an entire periphery image can be associated with the entire periphery image, for example, at a reference position such as the pupil center. Also in the second imaging apparatus, each position on the second front image can be associated with an acquisition position of sectional information. Therefore, any line of longitude (section) passing through the reference position can be specified for each position on the entire periphery image via the first front image which is captured in advance by the first imaging apparatus and is associated with the entire periphery image and the second front image which is used as an observation image in the second imaging apparatus. Therefore, for example, with respect to a specific ACA tissue detected from the entire periphery image or a position designated by an examiner in the entire periphery image, a position of the line of longitude passing through the tissue or the position can be specified on the second front image, and thus the second imaging apparatus controls driving of an optical system or an alignment drive unit so as to perform an operation of acquiring sectional information for the specified line of longitude on the second front image.

### [Examples]

Hereinafter, Examples will be described. First, an Example corresponding to the first embodiment will be described. As illustrated in Fig. 1, an ophthalmological system 1 related to the present example includes an angle imaging apparatus 100 (the "first imaging apparatus" in the first embodiment), an anterior ocular segment OCT 200 (the "second imaging apparatus" in the first embodiment), and a computer 300 (the "computer" in the first embodiment).

The angle imaging apparatus 100 captures a reflected image of an ACA of a subject eye. The angle imaging apparatus 100 of the present example images a part of the ACA entire periphery, and an entire periphery image is generated as a result of combining a plurality of reflected images captured at different positions with each other.

The anterior ocular segment OCT 200 scans the anterior ocular segment with measurement light so as to acquire OCT data of the anterior ocular segment. In the anterior ocular segment OCT 200, a scanning range of the measurement light includes the ACA (and the periphery thereof), and sectional information is acquired as the OCT data.

### <Angle imaging apparatus>

With reference to Fig. 2, a description will be made of a schematic apparatus configuration of the angle imaging apparatus 100. In the drawings including Fig. 2, an X direction is set to a leftward-and-rightward direction, a Y direction is set to an upward-and-downward direction, and a Z direction is set to a front-and-rear direction.

The angle imaging apparatus 100 has an imaging optical axis L2 tilted with respect to a fixation optical axis L1. The imaging optical axis L2 is disposed along an angle division line of the ACA in a state in which alignment is appropriate. The angle imaging apparatus 100 receives reflected light from an ACA region along the imaging optical axis L2, and thus captures a reflected image of the ACA.

The angle imaging apparatus 100 includes at least an imaging optical system 100a and a control unit 170. The imaging optical system 100a is an optical system capturing a reflected image of the ACA. The control unit 170 controls the entire operation of the apparatus. In the present example, the control unit 170 is also used as an image processor in the angle imaging apparatus 100. For example, a reflected image captured by the imaging optical system 100a is processed by the control unit 170, and an entire periphery image is formed as a result thereof.

The imaging optical system 100a includes at least an objective reflection unit 140, a switching unit 130, and an imaging element 160. The angle imaging apparatus 100 may further include a light source 110 and a focus adjustment unit 150.

The light source 110 is a light source of illumination light applied to the ACA. In the present example, the light source 110 emits visible light. In the following description, it is assumed that the light source 110 can emit at least light (for example, white light) of a plurality of colors having different wavelength ranges in order to obtain an ACA image as a color image.

The illumination light from the light source 110 is reflected by a beam splitter 120 so as to be directed toward the switching unit 130. In this case, reflected light of the illumination light is guided to the switching unit 130 along the fixation optical axis L1. The beam splitter 120 splits a light projection path from a light reception path in the imaging optical system 100a. As the beam splitter 120, a half mirror or a hollow mirror may be used.

The switching unit 130 is a unit which displaces an imaging position of a reflected image in the entire periphery of the ACA. Since the switching unit 130 is provided, reflected images of the ACA at a plurality of imaging positions in the entire periphery of the ACA can be captured. In the present example, the switching unit 130 includes mirrors 131 and 132, and a drive source (not illustrated) such as a motor. The switching unit 130 is driven on the basis of a control signal from the control unit 170, and, as a result, the imaging optical axis L2 is rotated about the fixation optical axis L1.

The two mirrors 131 and 132 illustrated in Fig. 2 are disposed in parallel to each other, and shift the optical path center of illumination light by a predetermined gap with respect to the fixation optical axis L1. The mirrors 131 and 132 are rotated centering on the fixation optical axis L1 by the drive source (not illustrated), and thus a position of an irradiation optical path of illumination light from the switching unit 130 to the objective reflection unit 140 is displaced.

The objective reflection unit 140 has a reflection surface for bending illumination light toward the fixation optical axis L1 side. An optical axis of the illumination light reflected by the reflection surface is bent to be greatly tilted with respect to the fixation optical axis L1, so as to be guided to the outside of the apparatus. In this case, the optical axis guided to the outside of the apparatus is used as the imaging optical axis L2.

In the present example, the objective reflection unit 140 has a plurality of reflection surfaces formed side by side around the fixation optical axis L1. In the present example, a specific example of the objective reflection unit 140, a truncated conic prism having a regular polygon as a bottom surface is used. More specifically, the prism of which the bottom surface is a regular hexadecagon and which has sixteen side surfaces is used. In the present embodiment, the reflection surfaces directed toward the fixation optical axis L1 in the respective directions of 0 degree angle, 22.5 degree angle, 45 degree angle, 67.5 degree angle, 90 degree angle, ..., and 337.5 degree angle when viewed from a subject eye E are disposed. Each angle is an angle with respect to the fixation optical axis L1. For convenience of description, 0 degree angle is set on the horizontal plane (more specifically, the right side on the horizontal plane when viewed from the angle imaging apparatus 100).

The control unit 170 selects one of the sixteen reflection surfaces as a surface to which the switching unit 130 guides the illumination light, and can thus guide the illumination light to an imaging position corresponding to each reflection surface in the ACA entire periphery.

Reflected light from the ACA region is returned through the objective reflection unit 140 and the switching unit 130, so as to be guided to the beam splitter 120. The illumination light which passes through the beam splitter 120 toward the imaging element 160 side is received by the imaging element 160 via a focus lens 151. As a result, a reflected image of the ACA as illustrated in Fig. 3 is captured.

The focus lens 151 is a part of a focus adjustment unit of the angle imaging apparatus 100. The angle imaging apparatus 100 is provided with a drive unit 152 which moves the focus lens 151 along the optical axis. The drive unit 152 may include, for example, a linear actuator. A position of the focus lens 151 is adjusted according to a desired tissue of the ACA, and thus an image of the ACA including the desired tissue is captured on the basis of a light reception signal from the imaging element 160. In the present example, a focus is adjusted by moving the focus lens 151, and this is only an example. For example, a focus may be adjusted by moving the imaging element 160 in the optical axis direction.

In the present example, the control unit 170 causes reflected images to be captured a plurality of times in different focus states at each imaging position. Consequently, the reflected images in which focus states are different from each other are generated at each imaging position. The control unit 170 generates a multi-focus image which is in focus at each position, at each imaging position. In a plurality of reflected images in which imaging positions are the same as each other and focus states are different from each other, an image of which a focus state is most favorable is determined for each region into which the reflected image is subdivided, by the control unit 170. In this case, the control unit 170 may perform a positioning process on the respective reflected images. Subdivision regions can be associated with each other in the respective reflected images.

In the present example, the control unit 170 subjects the multi-focus images generated at the respective imaging positions in the ACA entire periphery to a collage, so as to generate an entire periphery image (refer to Fig. 4). The generated entire periphery image may be stored in a memory (not illustrated) of the angle imaging apparatus 100.

A plurality of entire periphery images in which focus states are different from each other may be first generated, and then a may further include of the ACA entire periphery may be generated on the basis of the plurality of entire periphery images in which focus states are different from each other.

### <Anterior ocular segment OCT>

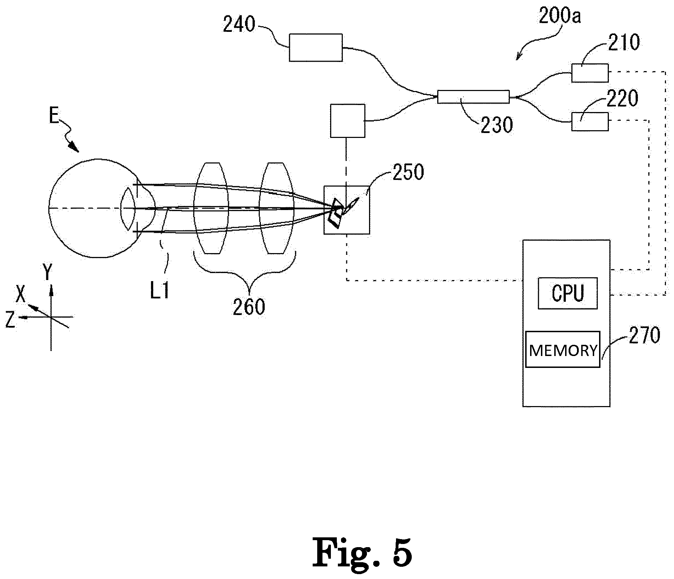

As illustrated in Fig. 5, the anterior ocular segment OCT 200 of the present example substantially telecentrically applies measurement light with respect to the fixation optical axis L1, and acquires OCT data of the anterior ocular segment in which a direction parallel to the eye axis of the subject eye E is a depth direction. The anterior ocular segment OCT 200 may be a spectral domain type OCT (SD-OCT), and may be a wavelength-swept OCT.

The anterior ocular segment OCT 200 includes at least an OCT optical system 200a and a control unit 270. The control unit 270 controls an operation of the entire apparatus. In the present example, the control unit 270 is also used as an image processor in the anterior ocular segment OCT 200.

The OCT optical system 200a may include a light source 210, a detector 220, a coupler (splitter) 230, and a reference optical system 240. The coupler 230 in the OCT optical system 200a divides light from the light source 210 into measurement light and reference light. An interference signal between return light of the measurement light applied to the association and the reference light is detected by and output from the detector 220. OCT data of the anterior ocular segment is obtained on the basis of the interference signal output from the detector 220. In the present example, a scanning range of the measurement light is assumed to include the ACA of the subject eye. In other words, the anterior ocular segment OCT may acquire OCT data of the ACA by scanning the ACA with the measurement light.

The OCT optical system 200a includes an optical scanner 250 and an objective lens 260 between the coupler 230 and the subject eye E. The optical scanner 250 is disposed in a focus of the objective lens 260 or the vicinity thereof, and thus the measurement light is applied in substantially parallel to the fixation optical axis L1. Scanning with the measurement light is performed on the anterior ocular segment according to an operation of the optical scanner 250.

Each unit of the anterior ocular segment OCT 200 is connected to the control unit 270, and is operated on the basis of a control signal from the control unit 270. The interference signal output from the detector 220 is processed by the control unit 270 so as to be acquired as OCT data. A tomographic image and a three-dimensional image of the ACA are generated on the basis of the OCT data.

### <Computer>

In the present embodiment, the computer 300 associates an entire periphery image which is a reflected image of the ACA entire periphery with OCT data of the ACA, and displays the entire periphery image and a sectional image based on the OCT data on a monitor 340 in association with each other.

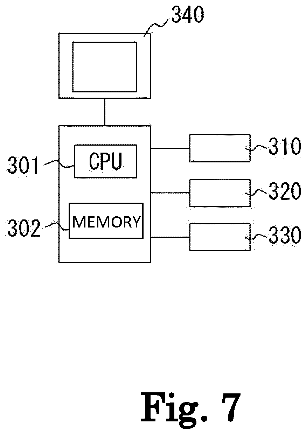

As illustrated in Fig. 7, the computer 300 includes at least a CPU (an example of a calculation processing unit) 301, a memory (storage device) 302, and an input/output port 310. The CPU 301 is a processor which performs a principal operation in the computer 300.

The memory 302 is a storage device storing various information, and includes at least a volatile storage medium (for example, a register, a cache, and a RAM) temporarily storing data, and a nonvolatile storage medium (for example, a ROM, an HDD, and a flash memory) storing a control program and fixed data. In the present embodiment, of the two media, an ophthalmological image processing program may be stored in the nonvolatile storage medium. The nonvolatile storage medium may include a repeatedly rewritable storage medium. In this case, data obtained as a result of execution of the ophthalmological image processing program may be stored in the rewritable nonvolatile storage medium.

The input/output port 310 electrically connects the computer 300 to an external apparatus. In the present example, in addition to the angle imaging apparatus 100 and the anterior ocular segment OCT 200, an externally attached storage medium 320, an operation unit 330, and the monitor 340 are connected to the computer 300 via the input/output port 310.

The operation unit 330 is a user interface used for an examiner to input an operation. A mouse or a keyboard may be used as the operation unit 330. Various images and information regarding the angle imaging apparatus 100 and the subject eye E are displayed on the monitor 340. Display control of the monitor 340 is performed by the CPU 301. In other words, in the present embodiment, the CPU 301 is also used as a display control unit.

### <Operation description>

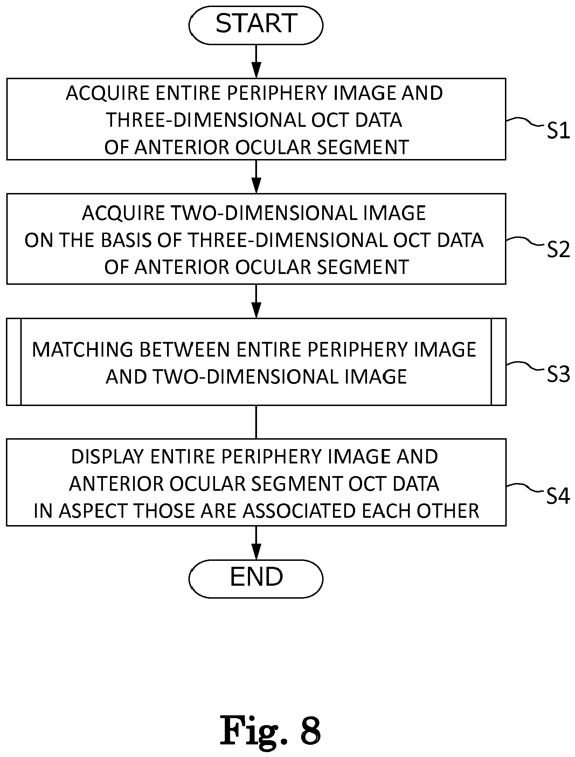

Hereinafter, with reference to a flowchart in Fig. 8, a description will be made of an operation of the computer 300 performed on the basis of the ophthalmological image processing program.

In the present example, it is assumed that an entire periphery image is generated in advance by the angle imaging apparatus 100, and OCT data of the entire anterior ocular segment including the ACA is generated in advance by the anterior ocular segment OCT 200. The entire periphery image is assumed to be a multi-focus image.

First, the computer 300 acquires an entire periphery image of an ACA of a subject eye and OCT data of the anterior ocular segment of the ACA of the subject eye (S1). Herein, the computer 300 the entire periphery image and the anterior ocular segment OCT data respectively transmitted from the angle imaging apparatus 100 and the anterior ocular segment OCT 200, in the memory via the input/output port 310.

Next, the computer 300 associates the entire periphery image with the anterior ocular segment OCT data acquired in advance through the process in S1 with each other through a matching process therebetween (S2 and S3).

Specifically, in the present example, a two-dimensional image of when a three-dimensional image is viewed along the optical axis L2 of the angle imaging apparatus 100 (S2), and a matching process is performed between the two-dimensional image and the entire periphery image (S3).

In the present example, the two-dimensional image is acquired as a projection layer of a surface layer of the three-dimensional image by subjecting the surface layer of the three-dimensional image to projection conversion with respect to a plane orthogonal to the optical axis L2 of the angle imaging apparatus 100. Angle information between a depth direction (herein, the Z direction) of OCT data in the anterior ocular segment OCT 200 and the imaging optical axis L2 in the angle imaging apparatus 100 is necessary during the projection conversion, but may be stored in the memory 302 as known data based on design values of the angle imaging apparatus 100 and the anterior ocular segment OCT 200. A method of projecting any point on a three-dimensional space onto any plane is well known, and thus a detailed description of an algorithm for generating a projection image will be omitted. The two-dimensional image is not limited to a projection image, and various OCT front images may be used. For example, the two-dimensional image may be an image of a horizontal section located at a predetermined depth in the optical axis L2 direction, and may be an image obtained by integrating respective layers of the three-dimensional image in the optical axis L2 direction.

In this case, in the projection image, a feature part based on undulations of a tissue which can be specified in the three-dimensional image may be projected onto the projection image. For example, a position or a shape of a feature part which is not reflected in the projection image in a case where the three-dimensional image is merely projected without being changed may be specified and emphasized on the projection image. The position of the feature part specified and emphasized on the projection image may be used for matching with the entire periphery image. The feature point based on undulations of a tissue may be, for example, a bottom of the ACA or an undulation of the iris.

Next, matching is performed between the projection image and the entire periphery image. In the matching, a common feature which is generated as an image in both of the projection image and the entire periphery image may be used. Phase images of the projection image and the entire periphery image may be generated, and matching may be performed between the phase images. In the present embodiment, a positional relationship in which an interrelation between the projection image and the entire periphery image is the maximum is retrieved while moving and rotating the entire periphery image with respect to the projection image, and the three-dimensional image is associated with the entire periphery image in the positional relationship in which the interrelation is the maximum.

The matching may be rigid body registration, and may be non-rigid body registration. An image provided for the rigid body registration may be subjected to linear conversion (enlargement and reduction, parallel movement, shearing, and rotation).

In the present example, since the entire periphery image is a multi-focus image, and defocus is suppressed in each part, matching with the projection image is easily performed with high accuracy.

Next, the computer 300 displays the entire periphery image and the three-dimensional image on the monitor 340 in a display aspect in which the images are associated with each other (S4). Figs. 9 and 10 illustrate an example of a display aspect.

In the example illustrated in Fig. 9, the entire periphery image and the three-dimensional image are displayed at different positions separately and simultaneously. An indicator 400 is disposed to overlap the entire periphery image at a position corresponding to the three-dimensional image, and thus a correspondence relationship between the entire periphery image and the three-dimensional image is indicated. An examiner can understand the correspondence relationship between the entire periphery image and the three-dimensional image on the basis of the indicator 400. A state of the ACA at the position of the indicator 400 can be observed on the basis of both of the entire periphery image and the three-dimensional image.

In a case where a three-dimensional image for the ACA entire periphery is acquired in advance, the indicator 400 on the entire periphery image may be changed on the basis of an operation on the operation unit 330, and the three-dimensional image displayed on the monitor 340 may be switched on the basis of an operation of changing a position of the indicator 400 such that the three-dimensional image is displayed at a position corresponding to the position of the indicator 400.

In the example illustrated in Fig. 9, position indicators are displayed with respect to the entire periphery image. The position indicators mentioned here are information for specifying positions of respective regions of the ACA entire periphery. In the example illustrated in Fig. 9, four letters such as "N", "S", "T", and "I" are displayed as the position indicators. In a case of the right eye, the position indicators are displayed by the letter "N" with respect to an image at the imaging position of 0 degree angle (that is, an image at the imaging position on the nose side), the letter "S" with respect to an image at the imaging position of 90 degree angle (that is, an image at the imaging position of the upper side), the letter "T" with respect to an image at the imaging position of 180 degree angle (that is, an image at the imaging position of the temple side), and the letter "I" with respect to an image at the imaging position of 270 degree angle (that is, an image at the imaging position of the lower side). The letters "N", "S", "T", and "I" respectively stand for Nasal, Superior, Temporal, and Inferior. The same position indicators may also be displayed in a display region of the three-dimensional image.

In the present example, the three-dimensional image is displayed, but this is only an example, and a two-dimensional sectional image may be displayed in a display aspect of being associated with an entire periphery image. In this case, in a case where any cut line is designated o the entire periphery image, a two-dimensional sectional image indicating a section based on the cut line may be displayed on the monitor 340.

As illustrated in Fig. 9, in a case where the three-dimensional image is displayed, the three-dimensional image may be rotated in any direction, for example, on the basis of an operation of an examiner. Consequently, the examiner can multilaterally observe a stereoscopic shape of a tissue of the ACA. In a case where a position is designated with respect to one of the entire periphery image and the three-dimensional image (more specifically, a surface layer thereof) on the basis of an operation on the operation unit 330 performed by the examiner, the indicator may overlap a position corresponding to the designated position on the other image.

As another example of the display aspect in which the entire periphery image is associated with the three-dimensional image, an image in which the entire periphery image is merged into the surface layer of the three-dimensional image may be displayed. For example, in the present example, an entire periphery image having a positional relationship in which an interrelation with a projection image is the maximum is subjected to inverse projection conversion with respect to the optical axis L2, and is merged into the surface layer of the three-dimensional image. As a result of the merging, as illustrated in Fig. 10, information regarding an ACA surface included in the entire periphery image is reflected in a three-dimensional model. Consequently, the examiner can more favorable understand a three-dimensional structure of the ACA.

### <Modification examples>

As mentioned above, the present disclosure has been described on the basis of the embodiment, but may be variously modified.

### <Modification example: association of reflected image with sectional information in part of ACA entire periphery>

For example, in the above-described embodiment, the computer associates an entire periphery image of the ACA with sectional information, but is not necessarily limited thereto. For example, the computer may associate a reflected image of the ACA acquired as an imaging result in the first imaging apparatus with sectional information acquired as an imaging result in the second imaging apparatus. The reflected image mentioned here may be either one of an entire periphery image of the ACA and a reflected image of a part of the ACA entire periphery. An association method and the type of sectional information may employ other description of the present disclosure.

### <Modification example: PS-OCT>

For example, the anterior ocular segment OCT 200 described in the Example may have a configuration of a PS-OCT. The PS-OCT indicates a polarization sensitive OCT, and may acquire at least one of birefringence (retardation), a polarization axis (axis orientation), and diattenuation of the inside of a subject.

As an example of the PS-OCT, a configuration example of a PS-SS-OCT will be described. The SS-OCT indicates a swept source OCT using a wavelength swept light source. In this case, the anterior ocular segment OCT 200 may include a first division member and a second division member. The first division member generates a plurality of interference light beams having a predetermined phase difference. The second division member generates a plurality of interference light beams having different polarization directions. A first channel may balance-detect two interference light beams which have an identical polarization direction (horizontal polarization in the present specification) and have a phase difference therebetween. A second channel may balance-detect two interference light beams which have an identical polarization direction (vertical polarization in the present specification) and have a phase difference therebetween. A polarization direction of the interference light detected by the first channel is different from a polarization direction of the interference light detected by the second channel. In this case, the anterior ocular segment OCT 200 may acquire polarization characteristic data of the ACA in a state in which the influence of a characteristic difference between the respective channels is reduced, on the basis of an interference signal at each wavelength, having a vertical polarization component and a horizontal polarization component. By using the PS-OCT, a feature part (for example, in the ACA, a pigment or a filamentous tissue) which is regarded not to be able to be detected from intensity OCT data can be detected through processing of the polarization characteristic data. By using the PS-OCT, the Schlemm's canal can also be expected to be favorably detected. However, the Schlemm's canal may be detected from the intensity OCT data. Other feature parts which are regarded not to be able to be detected from the intensity OCT data in the current state are expected to be detected from the intensity OCT data with the future advance of an analysis technique.

### <Modification example of objective reflection unit of angle imaging apparatus>

For example, the reflection surface formed in the objective reflection unit 140 of the angle imaging apparatus 100 is not necessarily required to be divided into a plurality of parts, and may be formed of a series of curved surfaces. The objective reflection unit 140 is not necessarily required to be a prism, and may be, for example, a reflection mirror. In a case of the reflection mirror, the reflection mirror may be a tubular polygon mirror or curved mirror having a reflection surface on an optical axis side.

### <Modification example of switching unit of angle imaging apparatus>

The switching unit 130 of the angle imaging apparatus 100 switches an imaging range in the ACA entire periphery by rotating a part of the imaging optical system, but is not necessarily limited thereto. For example, instead of rotating the imaging optical axis tilted with respect to the fixation optical axis about the fixation optical axis, the switching unit may be configured to guide fixation such that a direction of a visual line of a subject eye is greatly displaced, may be configured to adjust a three-dimensional positional relationship between a subject eye and the apparatus, and may have a combined configuration thereof.

## Claims

1. An ophthalmological image processing program, when executed by a processor of a computer, causing the computer to perform:
an acquisition step of acquiring an entire periphery image being a reflected image of an entire periphery of an ACA (Anterior Chamber Angle) of a subject eye as an imaging result of the ACA by a first imaging apparatus, and acquiring sectional information of the ACA as an imaging result of an anterior ocular segment of the subject eye by a second imaging apparatus; and
an association step of associating the entire periphery image with the sectional information in regard to a positional relationship.

2. The ophthalmological image processing program according to claim 1,
wherein, in the acquisition step, the processor acquires, as the entire periphery image, a color image in which visible light including a plurality of different wavelength ranges is used as illumination light.

3. The ophthalmological image processing program according to claim 1 or 2,
wherein the second imaging apparatus is an OCT apparatus that acquires OCT data of the anterior ocular segment,
in the acquisition step, the processor acquires three-dimensional OCT data of the anterior ocular segment as the sectional information, and
in the association step, the processor associates the three-dimensional OCT data with the entire periphery image in regard to a positional relationship.

4. The ophthalmological image processing program according to claim 3,
wherein, in the acquisition step, the processor acquires three-dimensional motion contrast data including a blood vessel region of the anterior ocular segment as the three-dimensional OCT data, and
in the association step, the processor associates the three-dimensional motion contrast data with the entire periphery image in regard to a positional relationship.

5. The ophthalmological image processing program according to claim 4,
wherein, in the acquisition step, the processor further acquires three-dimensional intensity OCT data corresponding to the three-dimensional motion contrast data, and
in the association step, the processor associates the three-dimensional intensity OCT data with the entire periphery image to indirectly associate the three-dimensional motion contrast data with the entire periphery image.

6. The ophthalmological image processing program according to any one of claims 3 to 5,
wherein, in the association step, the processor performs a matching process between a three-dimensional image based on the three-dimensional OCT data and the entire periphery image to associate the three-dimensional OCT data with the entire periphery image in regard to a positional relationship.

7. The ophthalmological image processing program according to any one of claims 3 to 5,
wherein the first imaging apparatus projects and receives illumination light via an optical axis parallel to an angle division line in the ACA, and acquires the entire periphery image based on illumination light projected and received while changing a position of the optical axis with respect to a plurality of positions in the ACA entire periphery, and
in the association step, the processor acquires an OCT front image viewed along the optical axis of the first imaging apparatus based on the three-dimensional OCT data, and associates the three-dimensional OCT data with the entire periphery image in regard to a positional relationship by performing a matching process between the OCT front image and the entire periphery image.

8. The ophthalmological image processing program according to claim 1 or 2,
wherein the first imaging apparatus projects and receives illumination light via an optical axis parallel to an angle division line in the ACA, and acquires the entire periphery image based on illumination light projected and received while changing a position of the optical axis with respect to a plurality of positions in the ACA entire periphery,
in the acquisition step, the processor acquires a three-dimensional image based on a plurality of sectional information in which positions of sections are different from each other, and acquires a two-dimensional image in a case of viewing the three-dimensional image along the optical axis of the first imaging apparatus, and
in the association step, the processor associates the entire periphery image with the three-dimensional image by performing a matching process between the two-dimensional image and the entire periphery image.

9. The ophthalmological image processing program according to claim 7 or 8,
wherein the matching process is an image process performed based on an interrelation between images.

10. The ophthalmological image processing program according to claim 7 or 8,
wherein the matching process is pattern matching performed with using either of information regarding a shape of the ACA common to images and information regarding a feature part of the ACA common to images.

11. The ophthalmological image processing program according to any one of claims 6 to 10,
wherein, in the acquisition step, the processor acquires, as the entire periphery image, a multi-focus image being in focus on a plurality of kinds of different feature parts in a single image.

12. The ophthalmological image processing program according to claim 11,
wherein the multi-focus image is a combined image obtained through a combination of a plurality of the reflected images in which focus states of an imaging optical system in the first imaging apparatus are different from each other.

13. The ophthalmological image processing program according to any one of claims 1 to 4,
wherein the first imaging apparatus further captures a first front image being a front image of the anterior ocular segment during capturing of the entire periphery image,
the second imaging apparatus further captures a second front image being a front image of the anterior ocular segment during generation and acquisition of the sectional information, and
in the association step, the processor associates the entire periphery image with the sectional information through the first front image and the second front image.

14. The ophthalmological image processing program according to any one of claims 1 to 13, when executed by the processor of the computer, causing the computer to further perform:
a display control step of simultaneously displaying the entire periphery image and a sectional image on a monitor in an aspect that the entire periphery image is associated with the sectional image based on the sectional information in regard to a positional relationship.
